# EUROPEAN PATENT APPLICATION

(11) **EP 2 263 664 A1**
(43) Date of publication of application: **22.12.2010**
(21) Application number: 09160492.6
(22) Date of filing: 18.05.2009
(51) Int. Cl.: A61K 31/122, A61K 36/28, A61K 36/53, A61K 36/71, A61P 37/00

(54) **Opioid receptors stimulating compounds (thymoquinone, Nigella sativa) and food allergy**

(71) Applicant: Nestec S.A., 1800 Vevey (CH)
(72) Inventor: Nutten, Sophie, 1005, LAUSANNE (CH); Philippe, David, 1003, LAUSANNE (CH); Mercenier, Annick, 1066, Epalinges (CH); Duncker, Swantje, 1012, LAUSANNE (CH)
(74) Representative: Marquardt, Ulf

(57) **Abstract**

The present invention relates generally to the fields of food allergy and nutrition. It was found that the stimulation of opioid receptors could be used to treat or prevent food allergy. One embodiment of the present invention concerns hence the use of an opioid receptor stimulating compound like thymoquinone or plant extracts from Nigella sativa, Eupatorium ayapana, Satureja montana or Thymus for the preparation of a composition to treat or prevent food allergy.

## Description

The present invention relates generally to the field of food allergy and nutrition. It was found that the stimulation of opioid receptors could be used to treat or prevent food allergy. One embodiment of the present invention concerns hence the use of an opioid receptor stimulating compound for the preparation of a composition to treat or prevent food allergy.

Food allergies represent a significant health problem of our society today. Food allergies affect all age groups, but in particular children. Nowadays around 6 to 8 percent of all children suffer from at least one food allergy. Being allergic to certain products, such as wheat or cow's milk, for example, makes it difficult to provide the body with all required nutrients in sufficient amounts. Adults are slightly less affected than children, but still around 4 percent of all adults suffer from food allergies. Obviously, it is also important for adults to provide the body with all necessary nutrients to maintain a good health status.

A large number of patients knows or assumes that they are allergic to a particular food component, but cannot tell exactly, to which compound they are allergic. So they try to avoid food that causes allergic reactions on a trial and error basis.

Additional to patients with confirmed food allergy there is a large number of people, up to 35%, suffering from hypersensitivity to one or more food allergens (Rona,R.J. et al., 2007, J. Allergy Clin. Immunol. 120: 638-646).

But even if the exact food product that causes allergic reactions is known, accidental exposures to allergenic foods happen rather frequently. On average, over a period of two years, approximately 50 percent of all subjects with food allergy have at least one allergic reaction due to an accidental exposure, which can result in clinical symptoms.

A large number of episodes of food-induced anaphylaxis results in quite severe pathologies, in the worst case leading to the death of the patient.

In general patients are managing their food allergy by avoidance diets, in which the allergic person avoids all forms of the food she or he is allergic to. Individuals with very strong allergic reactions may even have to avoid all physical contact with the allergenic food, including touching or inhaling. This - of course -will have severe implications for the quality of life of the allergic person.

It would hence be desirable to have available a composition, which facilitates the life of a person or an animal that suffers from a food allergy.

It was consequently the object of the present invention to improve the state of the art and to provide a composition that allows it to treat or prevent food allergies, to reduce the symptoms of food allergies, and/or to increase the level of tolerance of the allergic patient to an allergenic food.

The present inventors were surprised to see that they could achieve this object by means of the independent claims. The dependent claims further develop the invention.

The present inventors have used a mouse model for food allergy and could show that compounds that stimulated at least one opioid receptor could be used to treat or prevent food allergies. Male Balb/c mice were sensitized by intraperitoneal injection of ovalbumin (OVA) together with aluminium potassium sulphate. The mice were then orally challenged via OVA ingestion, which resulted in clinical symptoms of food allergy, namely transient diarrhea.

It turned out that tested opioid receptor agonists achieved the object of the present invention. Opioid receptor agonists reduced the clinical symptoms and allergy associated immune parameters in the murine model tested.

Opioid receptor agonists are a well known class of compounds. They are reviewed for example in the scientific literature by Janecka A. et al., 2004, Curr. Top. Med. Chem.4(1):1-17.

Consequently, one embodiment of the present invention is the use of an opioid receptor stimulating compound (opioid receptor agonist) for the preparation of a composition to treat or prevent food allergy.

The present invention also relates to a composition comprising at least one opioid receptor stimulating compound for treating or preventing food allergy.

For the purpose of the present invention the terms "food allergy", "allergy" and "allergic" include "hypersensitivity" as defined by the European Academy of Allergy and Clinical Immunology (EAACI) as *causing objectively reproducible symptoms or signs, initiated by exposure to a defined stimulus tolerated by normal subjects,* and "allergy" as defined by the EAACI as *hypersensitivity reaction initiated by immunologic mechanisms* (Johansson,S.G., et al., 2001, A revised nomenclature for allergy. An EAACI position statement from the EAACI nomenclature task force, Allergy 56, 813-824). The kind of opioid receptor that is stimulated by the opioid receptor stimulating compound is not found to be critical for the purpose of the present invention. It is essential, however, that the opioid receptor stimulating compound stimulates at least one opioid receptor.

In one embodiment of the present invention the opioid receptor stimulating compound is a µ-receptor stimulating compound. The opioid receptor stimulating compound may alternatively or additionally be a κ-receptor stimulating compound and/or a δ-receptor stimulating compound.

Opioid receptor stimulating compounds may stimulate more than one opioid receptor. Combinations of different opioid receptor stimulating compounds may be used.

Opioid receptor stimulating compounds may be found in plants such as Japanese rice, tomato, potato, Indian rice and other foods, e.g., milk and milk based products. Opioid receptor stimulating compound enriched extracts of these plants or foods may be used for the purpose of the present invention.

For example, the opioid receptor stimulating compound may be thymoquinone (2-Isopropyl-5-methyl-1,4-benzoquinone) and/or a thymoquinone containing extract.

Thymoquinone is the following compound:

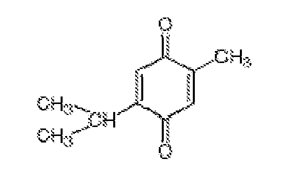

Thymoquinone has been used for medical purposes for more than 2,000 years. Typical applications were its use as antioxidant, anti-inflammatory, and antineoplastic medicines (Trang et al., Planta Med 1993;59:99; Hosseinzadeh et al., Phytomedicine 2004;11:56-64). Moreover, thymoquinone was recently found to inhibit tumor angiogenesis and tumor growth (Yi et al., 2008, Molecular Cancer Therapeutics 7, 1789-1796).

Thymoquinone may for example be found in plants such as *Nigella sativa, Eupatorium ayapana, Satureja montana* and/or *Thymus.* Particularly the seeds of *Nigella sativa* are a rich source of thymoquinone.

Consequently, the opioid receptor stimulating compound may be provided as component of a plant or a plant extract. For food applications it is particular preferred, if the opioid receptor stimulating compound is provided as an edible plant or an extract thereof. A material is considered "edible" if it is approved for human or animal consumption.

Plants include their fruits, seeds and roots as well as the rest of the plant. Edible plants have the advantage that they can be added to food products without having to purify the opioid receptor stimulating compound extensively. This will help to maintain the "NaturNes^{®}" in a product and - at the same time - saves costs and efforts for unnecessary purification steps. Also the use of chemically synthesized compounds can be avoided.

Hence, in one embodiment of the present invention, the opioid receptor stimulating compound is provided as *Nigella sativa, Eupatorium ayapana, Satureja montana* and/or *Thymus* or as an extract thereof.

A typical extract of *Nigella sativa* is a crude lipid extract. Such a crude lipid extract of *Nigella sativa* may contain about 0.22 weight-% thymoquinone.

The present inventors could show that the opioid receptor stimulating compounds reduce allergy associated immune parameters in general in a murine model for food allergy. This finding is independent of the exact allergen, so that the composition prepared by the use of the present invention will be effective against any kind of food allergy.

Hence, the type of food allergy is believed not to be critical for the purpose of the present invention. This has the advantage that the composition of the present invention can also be used under circumstances where the exact food derived allergens are not exactly known.

Consequently in one embodiment the food allergy is selected from the group consisting of dairy allergy, egg allergy, peanut allergy, tree nut allergy, sesame allergy, corn allergy, rice allergy, parsley allergy, buckwheat allergy, seafood allergy, shellfish allergy, soy allergy, wheat allergy or combinations thereof.

The composition of the present invention may also be used to treat or prevent the symptoms of food allergy. These symptoms may for example be selected from the group consisting of tissue swelling; itching of the mouth, throat, eyes and/or skin; nausea; vomiting; diarrhea; stomach cramps and/or abdominal pain; nasal congestion; wheezing; scratchy throat; shortness of breath; difficulty swallowing; or combinations thereof.

The composition of the present invention may be administered to any subject that is suffering from a food allergy or that is at risk of developing a food allergy.

For example, the composition may be intended for a human. Alternatively or additionally, the composition may also be intended for an animal, for example a pet animal.

Since children or infants are in particular affected by food allergies, the composition of the present invention may be intended for children and/or infants. According to the definitions appearing in Article 2 of the European Commission Directive 2006/141/EC of 22 December 2006 on infant formulae and follow-on formulae "Infants" are children under the age of 12 months and young children are children between 1 and 3 years of age.

The term "children" comprises the age groups from 1 to 14 years.

Of course, the compositions of the present invention may also be used for teenagers (15-17 years) or adults (18 years or older).

The form of the composition is also believed not to be crucial for the purpose of the present invention. Any composition is suited that allows it to administer at least one opioid receptor stimulating compound.

This may be a food composition, a pet food composition, a food product, a drink, a nutritional formula, an infant feeding formula, a nutraceutical, a food additive, and/or a medicament.

Food products according to the present invention include dairy products, such as fermented milk products, e.g. yoghurts, buttermilk, etc; ice creams; concentrated milk; milk; dairy creams; flavoured milk drinks; whey based drinks; toppings; coffee creamers; chocolate; cheese based products; soups; sauces; purees; dressings; puddings; custards; baby foods; nutritional formula, such as those for complete nutrition, e.g. for infants, children, teenagers, adults or the elderly; cereals and cereal bars.

Drinks include e.g. milk- or yoghurt based drinks, fermented milk, coffee, protein drinks, tea, energy drinks, soy drinks, fruit and/or vegetable drinks, fruit and/or vegetable juices.

The composition may be to be administered orally, enterally and/or parenterally, e.g., subcutaneously, or intramuscularly.

In therapeutic applications, the compositions are administered in an amount sufficient to at least partially cure or arrest the symptoms of the food allergies and its complications. An amount adequate to accomplish this is defined as "a therapeutically effective dose". Amounts effective for this purpose will depend on a number of factors known to those of skill in the art such as the severity and kind of the allergy and the weight and general state of the patient and genetic background.

In prophylactic applications, compositions according to the invention are administered to a patient susceptible to or otherwise at risk of developing a food allergy in an amount that is sufficient to at least partially reduce the risk of developing such a disorder. Such an amount is defined to be "a prophylactic effective dose". Again, the precise amounts depend on a number of patient specific factors such as the patient's state of health and weight and genetic background.

Generally, the compositions of the present invention are administered in a therapeutically effective dose and/or a prophylactic effective dose.

What these doses are can be easily determined by those skilled in the art.

Typically, for example, the opioid receptor stimulating compound is to be administered in a daily dose in the range of 0,1 mg/kg - 90 mg/kg body weight, preferably 1mg - 20 mg/kg body weight of the subject to be treated.

When the opioid receptor stimulating compound is to be administered as plant material, for example *Nigella sativa, Eupatorium ayapana, Satureja montana, Thymus* or combinations thereof, the plant material, for example *Nigella sativa, Eupatorium ayapana, Satureja montana,Thymus,* or combinations thereof may be administered in a daily dose in the range of 1 mg plant material /kg body weight - 50 g plant material /kg body weight, preferably in the range of 2 g plant material /kg body weight - 20 g plant material /kg body weight.

When the opioid receptor stimulating compound is to be administered as an extract of a plant material, for example an extract of *Nigella sativa, Eupatorium ayapana, Satureja montana* and/or *Thymus* or combinations thereof, the extract, for example of *Nigella sativa, Eupatorium ayapana, Satureja Montana, Thymus,* or combinations thereof may be administered in a daily dose in the range of 1 mg plant extract /kg body weight - 160 mg plant extract /kg body weight, preferably in the range of 6 mg plant extract /kg body weight - 80 mg plant extract /kg body weight.

The composition of the present invention may further comprise a protein source, a carbohydrate source and/or a lipid source.

For special clinical applications, in particular parenteral applications, it may be desirable to provide compositions which do not contain a carbohydrate source.

Since the allergen that triggers the allergic response is usually a food protein or a part thereof, the composition of the protein source in compositions intended for allergic patients requires particular attention. In general, the type of protein present in the composition should not trigger allergic reactions. Hence the protein sources used may vary depending on the type of allergy that is to be prevented or treated by the composition of the present invention.

Any suitable dietary protein may be used, for example animal proteins (such as milk proteins, meat proteins and egg proteins) or hydrolysates thereof; vegetable proteins (such as soy protein, wheat protein, rice protein, and pea protein) or hydrolysates thereof; mixtures of free amino acids; or combinations thereof. Milk proteins such as casein and whey, and soy proteins or hydrolysates thereof may be preferred for some applications. If the protein source is a milk protein or a milk protein fraction, it may be for example sweet whey, acid whey, α-lactalbumin, β-lactoglobulin, bovine serum albumin, acid casein, caseinates, α-casein, β-casein, γ-casein. Of course combinations of different protein sources may be used.

As far as whey proteins are concerned, the protein source may be based on acid whey or sweet whey or mixtures thereof and may include α- lactalbumin and β -lactoglobulin in whatever proportions are desired. Preferably however, in particular if the composition is an infant feeding formula, the protein source is based on modified sweet whey. Sweet whey is a readily available by-product of cheese making and is frequently used in the manufacture of infant formulas based on cows' milk.

The proteins may be intact or hydrolysed or a mixture of intact and hydrolysed proteins. It may be desirable to supply extensively or partially hydrolysed proteins (degree of hydrolysis between 2 and 20%). The hydrolysis step may digest potential allergenic food proteins. Consequently, the provision of hydrolyzed proteins may be beneficial for allergic patients or people at risk of developing an allergy.

If hydrolysed proteins are required, the hydrolysis process may be carried out as desired and as is known in the art. For example, a whey protein hydrolysate may be prepared by enzymatically hydrolysing the whey fraction in one or more steps.

If the composition of the present invention contains a protein source, then the amount of protein or protein equivalent in the composition is typically in the range of 1.6-7.5 g/100kcal of the composition.

In particular for nutritional formulas, the protein source should provide that the minimum requirements for essential amino acid content are met.

If the composition contains a carbohydrate source, the kind of carbohydrate to be used is not particularly limited. Any suitable carbohydrate may be used, for example sucrose, lactose, glucose, fructose, corn syrup solids, maltodextrins, starch and mixtures thereof. Combinations of different carbohydrate sources may be used. The carbohydrates may preferably provide 30% to 80% of the energy of the composition. For example, the composition may comprise a carbohydrate source in an amount of 9-18 g/100kcal of the composition.

Dietary fibre may be added as well. They may be soluble or insoluble and in general a blend of the two types is preferred. Suitable sources of dietary fibre include soy, pea, oat, pectin, guar gum, arabic gum, fructooligosaccharides, galacto-oligosaccharides, sialyl-lactose and oligosaccharides derived from animal milks. A preferred fibre blend is a mixture of inulin with shorter chain fructo-oligosaccharides.

If the composition contains a lipid source, the kind of lipid to be used is not particularly limited. If the composition includes a lipid source, the lipid source may provide 5% to 70% of the energy of the composition. Long chain n-3 and/or n-6 polyunsaturated fatty acids, such as DHA, ARA and/or EPA may be added. A suitable fat profile may be obtained using a blend of canola oil, corn oil, high-oleic acid sunflower oil and medium chain triglyceride oil. The composition may comprise a lipid source in an amount of 1.5-7 g/100kcal of the composition.

Those skilled in the art will understand that they can freely combine all features of the present invention described herein, without departing from the scope of the invention as disclosed. In particular, features described for the uses of the present invention may be applied to the compositions described in the present invention and vice versa.

Further advantages and features of the present invention are apparent from the following Examples and Figures.

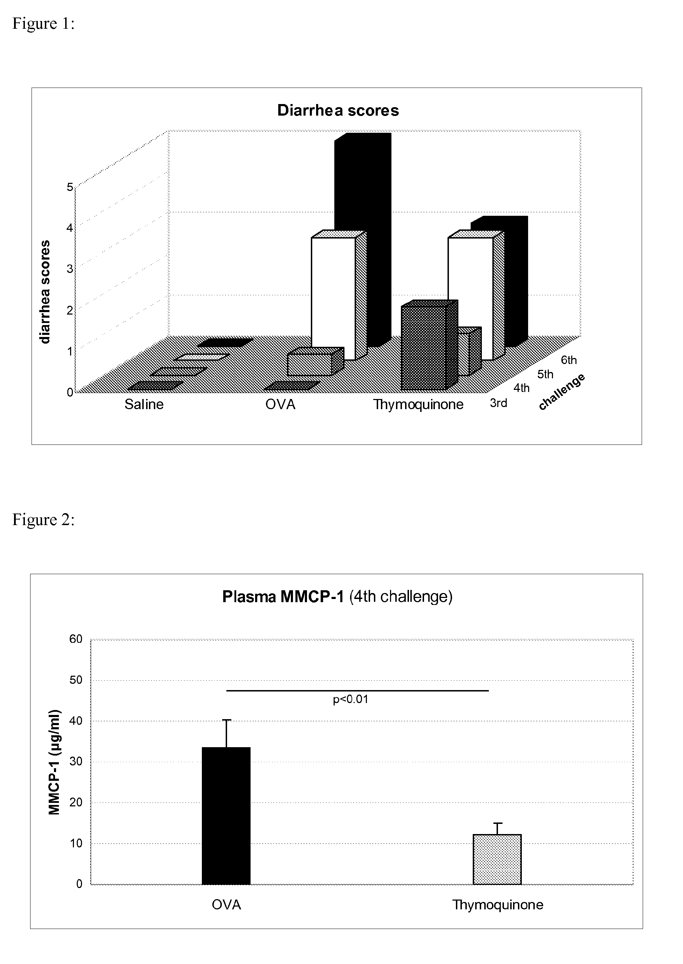

Figure 1 shows the diarrhea scores observed in OVA-sensitized mice challenged either with saline or OVA or challenged with OVA following treatment with thymoquinone. Results are displayed by the median (Median ± RobustSD: Saline = 0 ± 0, OVA = 5 ± 0; Thymoquinone = 3 ± 1.79).
Figure 2 shows the plasma concentration of MMCP-1 (at the 4th out of 6 challenges) in OVA sensitized mice challenged either with saline (value = 3.24 ± 0.27 ng/ml, data not shown) or OVA or challenged with OVA following treatment with thymoquinone. Results are displayed by the mean ± SEM in µg/ml plasma.

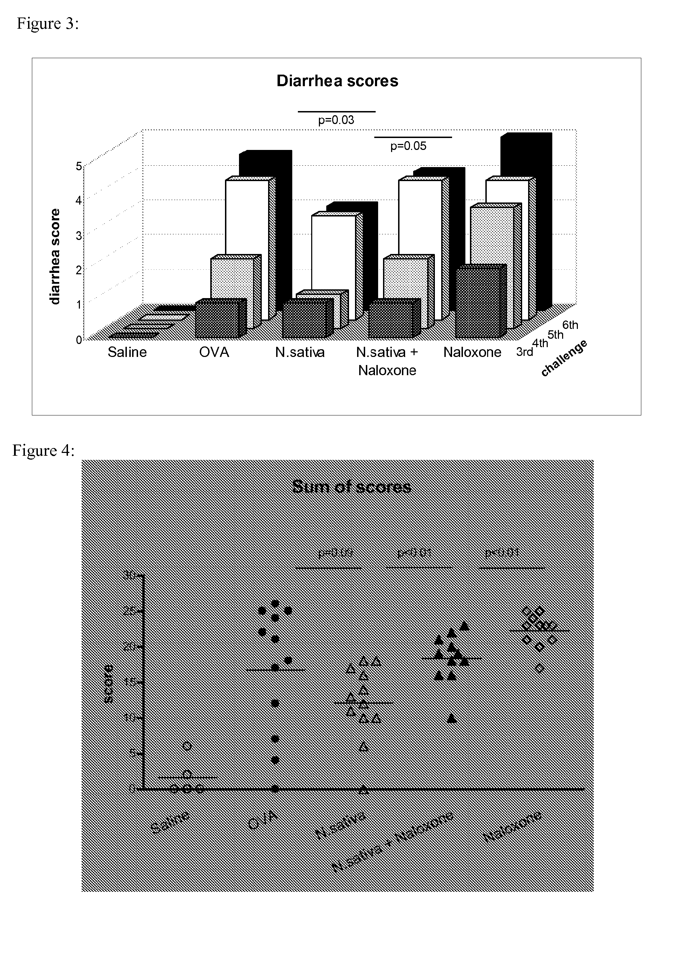

Figure 3 shows the diarrhea scores observed in OVA-sensitized mice challenged with saline (Saline) or ovalbumin (OVA), either treated with *Nigella sativa* extract (N. sativa), with *Nigella sativa* extract and the opioid receptor antagonist naloxone methiodide (N. sativa + Naloxone) or with naloxone methiodide alone (Naloxone). Results from the 3rd-6th challenge are shown and are expressed by the median (Median ± RobustSD 3^{rd} challenge:saline = 0 ± 0; OVA = 1 ± 1.2; *N. sativa* = 1 ± 1.2; *N. sativa*+Naloxone = 1 ± 0.6; Naloxone = 2 ± 1.2; 4^{th} challenge: saline = 0 ± 0; OVA = 2 ± 3.6; *N. sativa* = 1 ± 1.2; *N. sativa*+Naloxone = 2 ± 1.8; Naloxone = 3.5 ± 1.2; 5^{th} challenge: saline = 0 ± 0; OVA = 4 ± 0; *N. sativa* = 3 ± 1.2; *N. sativa*+Naloxone = 4 ± 0; Naloxone = 4 ± 0; 6^{th} challenge: saline = 0 ± 0.6; OVA = 4.5 ± 1.2; *N. sativa* = 3 ± 1.2; *N. sativa*+Naloxone = 4 ± 1.2; Naloxone = 5 ± 0).
Figure 4 shows the sum of diarrhea scores (3rd-6th challenge) observed in OVA-sensitized mice challenged with saline or ovalbumin, either treated with *Nigella sativa* extract, with *Nigella sativa* extract and the opioid receptor antagonist naloxone methiodide or with naloxone methiodide alone. Results are expressed as the individual values ± median.

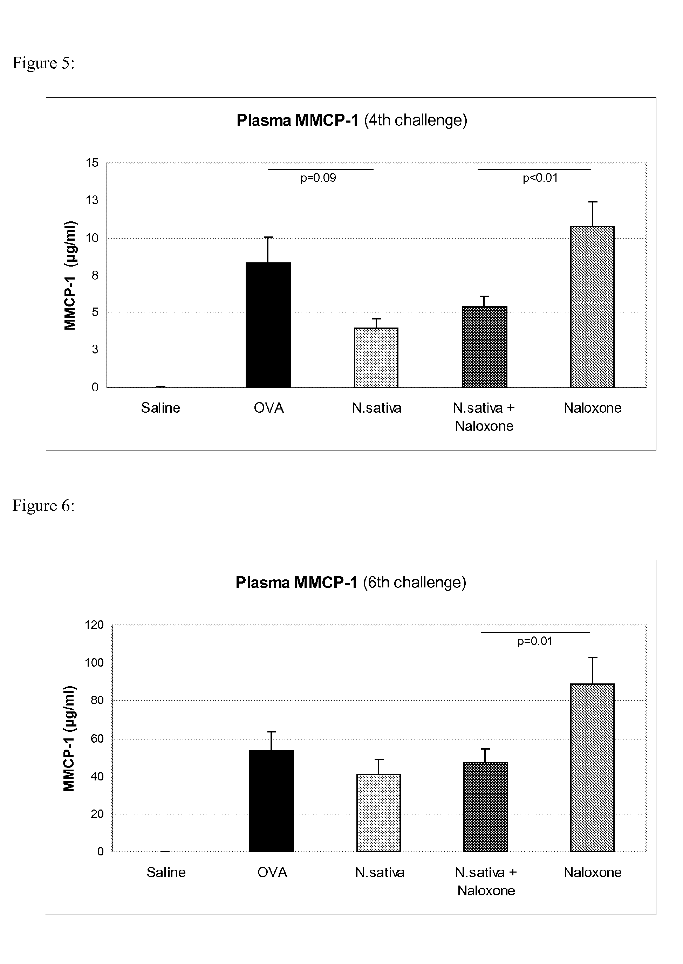

Figure 5 shows the plasma concentration of MMCP-1 (4th challenge) in OVA sensitized mice challenged with saline (value = 34 ± 2.45 ng/ml) or OVA either treated with *Nigella sativa* extract, *Nigella sativa* extract and naloxone methiodide or naloxone methiodide alone. Results are displayed by the mean ± SEM in µg/ml plasma.
Figure 6 shows the plasma concentration of MMCP-1 (6th challenge) in OVA sensitized mice challenged with saline (value = 80 ± 12.25 ng/ml) or OVA either treated with *Nigella sativa* extract, *Nigella sativa* extract and naloxone methiodide or naloxone methiodide alone. Results are displayed by the mean ± SEM in µg/ml plasma.

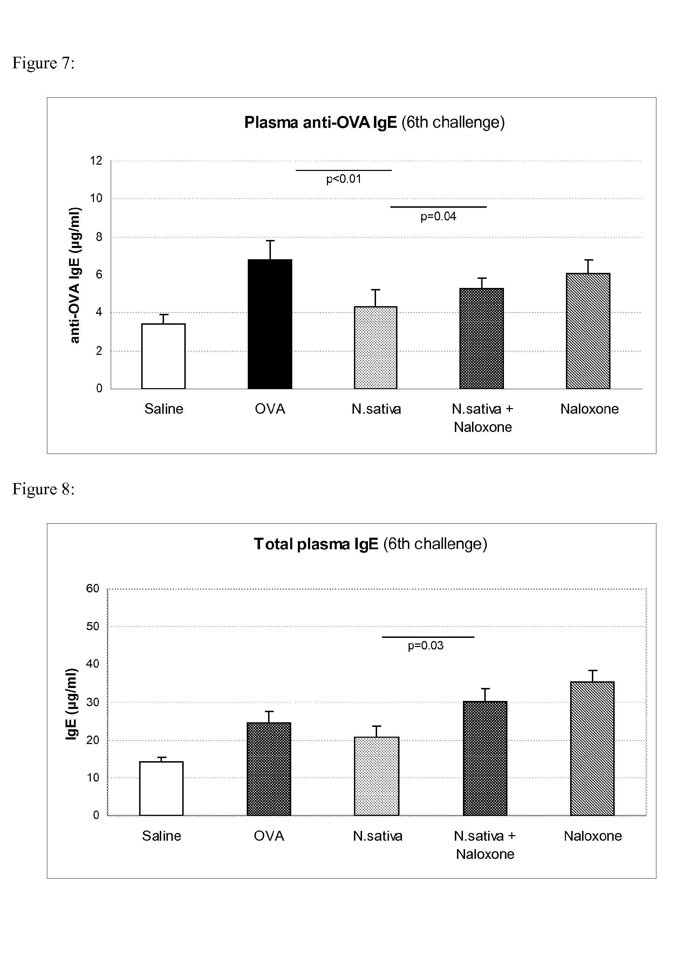

Figure 7 shows the plasma levels of OVA specific IgE (6^{th} challenge) in OVA sensitized mice challenged with saline (Saline) or OVA (OVA), either treated with *Nigella sativa* extract (*N. sativa*), *Nigella sativa* extract and naloxone methiodide (*N. sativa* + Naloxone) or naloxone methiodide alone (Naloxone). Results are expressed by the mean ± SEM.
Figure 8 shows the plasma levels of total IgE (6^{th} challenge) in OVA sensitized mice challenged with saline or OVA either treated with *Nigella sativa* extract, *Nigella sativa* extract and naloxone methiodide or naloxone methiodide alone. Results are expressed by the mean ± SEM.

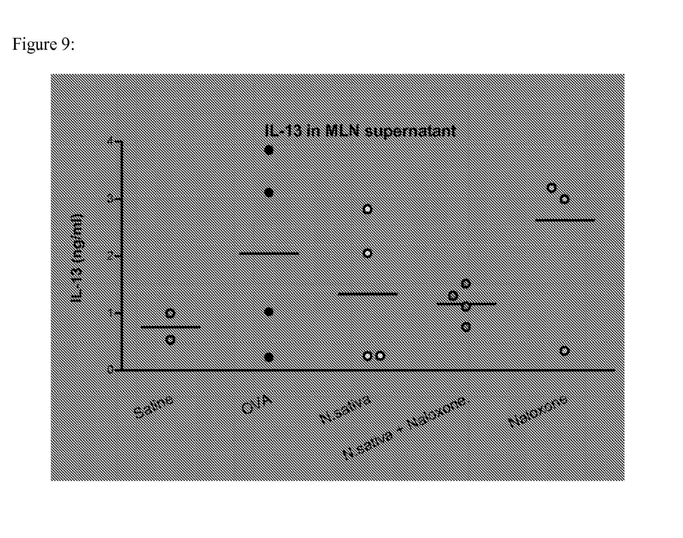

Figure 9 shows the concentration of IL-13 in supernatant from *ex vivo* OVA stimulated mesenteric lymphocytes from mice challenged with saline or OVA either untreated or treated with *Nigella sativa* extract or Naloxone methiodide alone or *Nigella sativa* extract and Naloxone methiodide (Results are shown for samples pooled from 2-3 mice).

### Examples:

### METHOD: MURINE MODEL OF OVA-INDUCED ALLERGIC DIARRHEA

Briefly, following sensitization (2 intraperitoneal injections of OVA and aluminium potassium sulphate at an interval of 14 days) adult male Balb/c mice were orally challenged with OVA for 6 times (days 28, 30, 33, 35, 37, 40) resulting in transient clinical symptoms (diarrhea) and changes of immune parameters (plasma concentration of total IgE, OVA specific IgE, mouse mast cell protease 1 (MMCP-1) as well as Th2 type cytokine production of *ex vivo* OVA re-stimulated mesenteric lymphocytes (MLN)). Substances, to be tested, were administered either by subcutaneous injection (thymoquinone and naloxone methiodide according to the literature) or by gavage (*Nigella sativa* extract).

### Effect of thymoquinone

### 1) Effect of thymoquinone on clinical symptoms

Mice treated with thymoquinone for 4 days prior to sensitization with OVA and during the challenge period significantly improve in their clinical symptoms observed after the 6th challenge when compared to non-treated mice (Fig. 1).

### 2) Effect of thymoquinone on immune markers

Mice that had received thymoquinone showed significantly lower amounts of mouse mast cell protease-1 (MMCP-1) in the plasma at the 4th challenge compared to non-treated OVA challenged controls (Fig. 2). At large the MMCP- 1 concentration in plasma correlates with the number of mast cells in the tissue which are known to be key players of diarrhea development in this mouse model (Brandt, E.B., et al., 2003, J. Clin. Invest 112, 1666-1677).

### Effect of Nigella sativa extract

One of the plants containing thymoquinone is *Nigella sativa* (Black Cumin, Black Seed). It has been used for centuries as spice and medicinal plant in Southern Europe, Northern Africa, Asia Minor and India. We tested crude lipid extract of *Nigella sativa* containing 0.22 weight-% thymoquinone in the murine model of OVA-induced allergic diarrhea.

### 1) Effect of Nigella sativa on clinical symptoms

Oral administration of *Nigella sativa* hexanic extract for 3 days before each sensitization and during the challenge period decreased clinical symptoms of mice sensitized and challenged with OVA (N. sativa) compared to controls (OVA) (Fig. 3). It also delayed the onset of symptoms with respect to the number of ovalbumin challenges needed to observe the first diarrhea symptoms (some OVA mice already display symptoms of diarrhea (score ≥ 3) at the 3rd challenge whereas 5 challenges were needed to observe the first diarrhea symptoms in *Nigella sativa* treated mice. Taking the sum of the individual scores from all 6 challenges, *Nigella sativa* treated mice showed a lower overall score compared with non-treated controls (OVA)(Fig. 4).

### 2) Effect of Nigella sativa on immune markers

Corresponding to the results in the clinical scores, feeding *Nigella sativa* showed a tendency to decrease plasma MMCP-1 levels following the 4th challenge compared to control mice (Fig. 5).

In line with the clinical symptoms, treatment with *Nigella sativa* extract significantly decreased allergy related immune markers like OVA specific IgE (Fig. 7) in the plasma, while leaving the total IgE unchanged (Fig. 8). OVA specific IgE is generated by plasma cells as a result of allergic reaction to ovalbumin, whereas at large total IgE is generated by plasma cells as result of a pro-allergic cytokine environment.

Results from *ex vivo* experiments with lymphocytes isolated from mesenteric lymph nodes, showed a tendency to lower the release of the allergy associated cytokine IL-13 following OVA re-stimulation of mesenteric lymphocytes from mice that had received *Nigella sativa* (Fig. 9).

Interestingly, the beneficial effects of *Nigella sativa* observed in mice with OVA-induced allergic diarrhea was -for most parameters- at least in part abolished by treatment with the peripheral opioid receptor antagonist naloxone methiodide, supporting a principle of action involving the opioid receptor pathway (Figures 4, 5, 8 and 9; comparison of N. sativa and N. sativa + Naloxone groups). Additionally, treatment with *Nigella sativa* reduced not only the clinical scores but also changed allergy related immune parameters, which suggests an additional effect beside the classical anti-diarrheic property (e.g. extended transit time).

All compounds that were tested positively for their effects against food allergies were shown to stimulate one or more opioid receptors.

For example thymoquinone showed binding to all three (µ-, κ-, δ-) opioid receptors known to date (Table 1). We therefore present here the data for synthetic thymoquinone as proof of principle.

**Table 1: Ligand displacement (%) from µ-, δ- and δ-opioid receptors by thymoquinone; values above 50% indicate significant ligand displacement and values between 20 and 50% weak to moderate displacement**

| | | | | |
|---|---|---|---|---|
| Substances tested (100µM) | CAS | µ-HOR % ligand displacement | κ-HOR % ligand displacement | δ-HOR % ligand displacement |
| Thymoquinone | 490-91-5 | 45 | 37 | 48 |

## Claims

1. Use of an opioid receptor stimulating compound for the preparation of a composition to treat or prevent food allergy.

2. Use in accordance with claim 1, wherein the opioid receptor stimulating compound is selected from the group consisting of µ-receptor stimulating compounds, κ-receptor stimulating compounds, δ-receptor stimulating compounds or combinations thereof.

3. Use in accordance with one of the preceding claims, wherein the opioid receptor stimulating compound is thymoquinone (2-Isopropyl-5-methyl-1,4-benzoquinone) and/or a thymoquinone containing extract.

4. Use in accordance with one of the preceding claims, wherein the opioid receptor stimulating compound is provided as a component of a plant or a plant extract, for example from *Nigella sativa, Eupatorium ayapana, Satureja montana, Thymus* or a combination thereof.

5. Use in accordance with one of the preceding claims, wherein the food allergy is selected from the group consisting of dairy allergy, egg allergy, peanut allergy, tree nut allergy, sesame allergy, corn allergy, rice allergy, buckwheat allergy, parsley allergy, seafood allergy, shellfish allergy, soy allergy, wheat allergy or combinations thereof.

6. Use in accordance with one of the preceding claims, to treat or prevent the symptoms of food allergy, for example symptoms selected from the group consisting of tissue swelling; itching of the mouth, throat, eyes and/or skin; nausea; vomiting; diarrhea; stomach cramps and/or abdominal pain; nasal congestion; wheezing; scratchy throat; shortness of breath; difficulty swallowing; or combinations thereof.

7. Use in accordance with one of the preceding claims, wherein the composition is to be administered to a subject selected from the group consisting of a human or a pet animal.

8. Use in accordance with one of the preceding claims, wherein the composition is selected from the group consisting of food compositions, food products, drinks, nutritional formulas, infant feeding formulas, nutraceuticals, food additives, medicaments.

9. Use in accordance with one of the preceding claims, wherein the composition is to be administered orally, enterally and/or parenterally.

10. Use in accordance with one of the preceding claims wherein the opioid receptor stimulating compound is to be administered in a daily dose in the range of 0,1 mg/ kg body weight - 90 mg/kg body weight, preferably 1 mg/kg body weight - 20 mg/kg body weight of the subject to be treated and/or wherein the opioid receptor stimulating compound is to be provided as *Nigella sativa* in a daily dose in the range of 1 mg *Nigella sativa* plant material /kg body weight - 50 g *Nigella sativa* plant material /kg body weight, preferably 2 g *Nigella sativa* plant material /kg body weight - 20 g *Nigella sativa* plant material /kg body weight of the subject to be treated and/or wherein the opioid receptor stimulating compound is to be provided as an extract of *Nigella sativa* in a daily dose in the range of 1 mg *Nigella sativa* plant extract /kg body weight - 160 mg *Nigella sativa* plant extract /kg body weight, preferably 6 mg *Nigella sativa* plant extract /kg body weight - 80 mg *Nigella sativa* plant extract/kg body weight of the subject to be treated

11. Use in accordance with one of the preceding claims wherein the composition further comprises a protein source in an amount of 1.6-7.5 g/100kcal of the composition.

12. Use in accordance with claim 11, wherein the protein source is hydrolyzed with a degree of hydrolysis (DH) in the range of between 2 and 20%.

13. Use in accordance with one of claims 11 or 12, wherein the protein source is milk protein or a milk protein fraction, for example sweet whey, acid whey, α-lactalbumin, β-lactoglobulin, bovine serum albumin, acid casein, caseinates, α-casein, β-casein, γ-casein.

14. Use in accordance with one of the preceding claims wherein the composition further comprises a carbohydrate source in an amount of 9-18 g/100kcal of the composition.

15. Use in accordance with one of the preceding claims wherein the composition further comprises a lipid source in an amount of 1.5-7 g/100kcal of the composition.
